Europäisches Patentamt

**European Patent Office**

**Office européen des brevets**

(19)

(11) Veröffentlichungsnummer: **0 009 198**
**A1**

(12) # EUROPÄISCHE PATENTANMELDUNG

(21) Anmeldenummer: 79103381.4

(51) Int. Cl.³: **G 01 N 33/54**

(22) Anmeldetag: **11.09.79**

(30) Priorität: **20.09.78 DE 2840847**

(43) Veröffentlichungstag der Anmeldung: **02.04.80**
**Patentblatt 80/7**

(84) Benannte Vertragsstaaten: **AT BE CH DE FR GB IT LU NL SE**

(71) Anmelder: **BEHRINGWERKE Aktiengesellschaft, Postfach 1140, D-3550 Marburg/Lahn (DE)**

(72) Erfinder: **Zahner, Horst, Dr., Lückweg 23, D-6331 Erda (DE)**
Erfinder: **Tandon, Aruna, Dr., Rudolf-Buchheim-Strasse 2, D-6300 Lahn-Giessen (DE)**
Erfinder: **Lämmler, Georg, Prof., Dr., Freiligrathstrasse 5, D-6300 Lahn-Giessen (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) **Verfahren zum Nachweis und zur Bestimmung komplementbindender Antikörper und Reagenz zur Durchführung dieses Verfahrens.**

(57) Zum Nachweis und zur Bestimmung eines komplementbindenden Antikörpers durch dessen Umsetzung mit einem Bindungspartner und Komplement wird ein Bindungspartner des komplementbindenden Antikörpers unlöslich gemacht, der unlöslich gemachte Bindungspartner mit dem komplementbindenden Antikörper sowie Serumkomplement zur Umsetzung gebracht, das Reaktionsprodukt mit einem Kupplungsprodukt eines Reaktionspartner des Serumkomplementes mit einem quantitativ bestimmbaren Markierungsmittel umgesetzt und schließlich das Markierungsmittel bestimmt. Hierfür kann ein Reagenz mit einem Gehalt an trägergebundenem Antigen, Serumkomplement und einem Komplementfaktorantikörper verbunden mit einem Markierungsmittel verwendet werden.

EP 0 009 198 A1

BEZEICHNUNG GEÄNDERT
siehe Titelseite

- 1 -

BEHRINGWERKE AKTIENGESELLSCHAFT HOE 78/B 011 Dr.Sn/Pa

## Verfahren zum Nachweis und zur Bestimmung komplementbindender Antikörper

Die Erfindung betrifft ein Verfahren zum Nachweis und zur Bestimmung komplementbindender Antikörper, vor allem in Körperflüssigkeiten, mittels eines enzymmarkierten Reaktanten.

Komplementbindende Antikörper sind solche Antikörper, die zum Beispiel bei Infektionskrankheiten entstehen und zwei haptophore Gruppen zur Verbindung mit dem Antigen und dem Komplement besitzen (Ambozeptoren). Der Nachweis komplementbindender Antikörper mit dem Komplementbindungstest ist ein häufig geübtes Verfahren in der Serologie. Insbesondere hat sich diese Methode in der Virologie und Parasitologie als Methode der Wahl zum indirekten Nachweis und zur ätiologischen Differenzierung von Infektionskrankheiten bewährt.

Der Komplementbindungstest ist bekanntlich schwierig zu handhaben und bedarf einer Reihe exakt aufeinander abgestimmter Reagenzien. Der eigentliche Nachweis der komplementbindenden Antikörper beruht auf der komplementabhängigen Hämolyse von Erythrozyten. Es ist deshalb erforderlich, zunächst das hämolytische System zu standardisieren, wonach aus der sekundär ablaufenden komplementabhängigen Hämolyse auf komplementbindende Antikörper und ihre Menge geschlossen werden kann. Das Verfahren ist sowohl zeit-

-2-                                    0009198

aufwendig als auch nur von besonders geschulten Kräften auszuüben. Es besteht demnach ein Bedarf, das Verfahren zum Nachweis und zur Bestimmung komplementbindender Antikörper zu vereinfachen.

Diese Aufgabe wird erfindungsgemäß dadurch gelöst, daß man die Reaktion an einer wasserunlöslichen Phase durchführt und statt des hämolytischen Systems ein in hoher Empfindlichkeit nachweisbares und quantitativ bestimmbares Markierungsmittel verwendet.

Gegenstand der Erfindung ist demnach ein Verfahren zum Nachweis und zur Bestimmung eines komplementbindenden Antikörpers durch dessen Umsetzung mit einem Bindungspartner und Komplement, dadurch gekennzeichnet, daß man einen Bindungspartner des komplementbindenden Antikörpers unlöslich macht, den unlöslich gemachten Bindungspartner mit dem komplementbindenden Antikörper sowie Serumkomplement zur Umsetzung bringt, das Reaktionsprodukt mit einem Kupplungsprodukt eines Reaktionspartners des Serumkomplements mit einem quantitativ bestimmbaren Markierungsmittel umsetzt und schließlich das Markierungsmittel bestimmt.

Der Bindungspartner für den komplementbindenden Antikörper ist üblicherweise das zu dem Antikörper korrespondierende Antigen. Es sind dies beispielsweise die viralen Antigene, gegen die der infizierte Organismus als Abwehrreaktion die interessierenden komplementbindenden Antikörper bildet. Beispielsweise sind hier zu nennen Cytomegalie, Herpes simplex- und Masern-Antigene, Influenza- und Parainfluenza-Antigene, Röteln-, Respiratory syncytial-, Mumps-, Ornithose-, Q-fieber-, Choriomeningitis- und Mycoplasma pneumoniae-Antigene, ferner Bruzella-, Toxoplasma- und Chagas-Antigene, um nur einige aus der Vielzahl der aufzuzählenden Antigene herauszugreifen.

Reaktionspartner dieses unlöslich gemachten Bindungspartners ist der komplementbindende Antikörper sowie das Komp-

0009198

lement selbst.

Das Komplement ist ein relativ labiler Bestandteil des Blutes, der nach heutigen Kenntissen aus mehr als 10 unterschiedlichen Faktoren besteht. Es ist bekannt, daß das Komplement bei Erwärmung auf 56° C wie auch bei längerem Stehen bei Zimmertemperatur seine Aktivität verliert. Frisches tierisches Serum ist in der Regel als Komplementquelle verwendbar. Im Sinne der Erfindung kommt menschliches Serum ebenso wie tierisches, vor allem Meerschweinchenserum in Frage.

Die spezifisch mit dem Komplement reagierende Komponente ist zweckmäßig ein gegen einen der Komplementfaktoren gerichteter Antikörper. Aus der experimentell abgeleiteten Vorstellung der Reaktionsweisen von komplementbindenden Antikörpern und Komplement werden diejenigen Antikörper gegen solche Komplementkomponenten bevorzugt, die als erste mit dem komplementbindenden Antikörper reagieren. Danach kommen als Reaktionskomponenten Antikörper gegen die Komplementfaktoren C 3 sowie C 5 bis C 9 in Frage. Antikörper gegen den Komplementfaktor C 3 werden besonders bevorzugt. Man verwendet hierzu eine geeignete Immunglobulinfraktion eines die betreffenden Antikörper enthaltenden Serums.

Als Markierungsmittel der vorgenannten Reaktionskomponenten, in der Regel der Antikörper gegen Komplementfaktoren $C_1$ bis $C_9$, kommen solche Substanzen in Frage, deren Nachweis in geringer Konzentration mit ausreichender Genauigkeit möglich ist. Bekannt sind hierfür vor allem Enzyme als markierende Substanzen. In einigen Fällen mag es vorteilhaft sein, statt dessen ein radioaktives Isotop, eine fluoreszierende Gruppe oder ein anderes Markierungsmittel zu verwenden. Das entsprechende Markierungsmittel ("Marker"), im Regelfalle das Enzym, ist mit dem Reaktionspartner des Komplements in der Weise verbunden, daß eine Abtrennung unter den Bedingungen der Reaktion nicht erfolgt. Das Markierungsmittel, z.B. das Enzym, kann in bekannter Weise nachgewiesen bzw. quantitativ bestimmt werden. Aus dem Ergebnis läßt sich auf die Anwesenheit bzw. die Menge des komplementbindenden Antikörpers schließen.

Im Sinne der Erfindung wird der Bindungspartner für den komplementbindenden Antikörper in unlöslicher Form in dem Bestimmungsverfahren eingesetzt. Dies kann durch physikalische Adsorption oder durch chemische kovalente Bindung auf der Oberfläche eines unlöslichen Trägers erfolgen. Vorteilhaft ist die Bindung an die Innenwand des Reaktionsgefäßes, in dem das Bestimmungsverfahren durchgeführt werden soll. Kunststoffgefäße aus Polyvinylchlorid oder Polystyrol sowie Träger (sogenannte Sticks) aus gleichen Materialien, die zur Reaktion in die Reaktionslösungen getaucht werden, sind wegen ihrer Eigenschaft der adsorptiven Bindung für die meisten Antigene besonders bevorzugt. Es können jedoch auch andere Materialien verwendet werden, die entweder eine Affinität für die Bindungspartner der Antikörper besitzen oder ihre kovalente Bindung ermöglichen. Besonders geeignet sind auch Mikrotitrationsplatten, wie sie z.B. aus DE-OS 26 33 283 bekannt sind.

Im Rahmen des erfindungsgemäßen Verfahrens wird zu diesem unlöslich gemachten Bindungspartner für komplementbindende Antikörper die Lösung gegeben, welche auf das Vorhandensein von komplementbindenden Antikörpern geprüft bzw. es wird der Träger in die Lösung gegeben, in welcher die komplementbindenden Antikörper quantitativ bestimmt werden sollen. Ferner wird dem Reaktionsgemisch Komplement zugesetzt. Eine geeignete Verdünnung des Komplements wird, falls dies im Einzelfall notwendig ist, durch in der biochemischen Analytik gebräuchliche Pufferlösungen erreicht. Besonders geeignet ist beispielsweise der von E.A. Kabat und M.M. Mayer, Experimental Immunochemistry, 2nd Edition, 1964, S. 149, hierfür vorgeschlage-

ne Barbituratpuffer, enthaltend $MgCl_2$ und $CaCl_2$. Nach einer Reaktionszeit von 1 bis 2 Stunden, vorzugsweise 2 Stunden und einer Temperatur von $4^O$ C bis $37^O$ C, vorzugsweise bei etwa $20^O$ C wird die feste Phase des Reaktionsgemisches von der flüssigen Phase abgetrennt und die feste Phase mehrfach gewaschen. Ausreichend und vorteilhaft erscheint ein 3 bis 5-maliges Waschen mit einer verdünnten Salzlösung, vorzugsweise einer Salzlösung mit einem Gehalt von weniger als 1 %. Besonders geeignet ist eine etwa 0,9 %ige Kochsalzlösung. Diese Salzlösung kann ggf. eine niedrige Konzentration eines Detergenz enthalten, welches die durch die Umsetzung entstandene feste Phase der spezifischen Reaktanten nicht irritiert. Besonders geeignet ist hier Polyoxyäthylensorbitanmonolaurat (Tween 20) in einer Konzentration $< 1$ %.

In einer folgenden Verfahrensstufe wird sodann die feste Phase mit dem markierten Reaktionspartner des Komplements umgesetzt. Hierfür wird eine vergleichbare Reaktionszeit wie bei der ersten Stufe als zweckmäßig angesehen, jedoch ist es nicht erforderlich, gleiche Reaktionszeiten zu wählen. Es kann sachdienlich sein, die Reaktionszeit der zweiten Stufe etwas länger vorzusehen. Die Entfernung nicht umgesetzten Materials erfolgt wiederum durch Waschen wie nach der ersten Umsetzungsstufe.

Da das fixierte Markierungsmittel, abhängig von der Menge des vorhandenen komplementbindenden Antikörpers, an die feste Phase gebunden ist, kann aus der gebundenen wie aus der freigebliebenen Menge des Markierungsmittels auf die Menge des komplementbindenden Antikörpers zurückgeschlossen werden. Erfoderlich ist lediglich die Bestimmung des gebundenen oder freien Markierungsmittels nach an sich bekannten Verfahren; bei den als Markierungsmittel bevorzugt verwendeten Enzymen die enzymatische Aktivität. Diejenigen Enzyme sind bevorzugt, deren Bestimmung auf einfache Weise durchgeführt werden kann.

0009198

In erster Linie sind dies die Enzyme, die eine spektrophotometrische oder kolorimetrische Bestimmung gestatten.
Ein besonderer Vorteil des erfindungsgemäßen Verfahrens
liegt demnach darin, daß eine Automatisierung der Bestimmung möglich ist. Somit ist auch die Bestimmung
komplementbindender Antikörper automatisierbar.

Enzyme, die als Markierungsmittel besonders bevorzugt
werden, sind Oxidasen und Hydrolasen, vor allem die alkalische Phosphatase, Glukoseoxidase, Peroxidase, um nur
einige beispielhaft zu nennen.

Die für das Verfahren notwendigen Reaganzien sind somit
der trägergebundene Bindungspartner des komplementbindenden Antikörpers, z.B. das Antigen; das Komplement; der
Antikörper gegen einen Komplementfaktor der mit einem
Markierungsmittel, vor allem einem Enzym verbunden ist;
sowie gegebenenfalls erforderliche Reaganzien zum Nachweis des Markierungsmittels z.B. das Enzymsubstrat.

Gegenstand der Erfindung sind somit auch die für das
Verfahren zum Nachweis und zur Bestimmung komplementbindender Antikörper erforderlichen Reagenzien sowie
deren Verwendung im Sinne der Erfindung.

Die Erfindung wird am nachstehenden Beispiel näher erläuert.

Beispiel:

In 0,3 ml-fassende Kunststoffgefäße, vertrieben von Dynatech Deutschland GmbH, Plochingen, wird jeweils 0,2 ml einer Chagas-Antigenlösung enthaltend 100 µg Antigen/ml gelöst in 0,05 M $Na_2CO_3$-Puffer vom pH 9,6 eingefüllt und über Nacht stehen gelassen.

Darauf werden die Gefäße entleert und drei mal mit physiologischer Kochsalzlösung unter Zusatz von 0,05% Tween 20 gewaschen. Diese Gefäße stellen danach das trägergebundene Antigen dar, das für das Verfahren zur Bestimmung komplementbindender Antikörper eingesetzt werden kann.

Für das Bestimmungsverfahren werden in die Gefäße jeweils 0,1 ml des zu prüfenden Serums in einer geeigneten Verdünnung und 0,1 ml eines 1:20 verdünnten normalen menschlichen Serums gefüllt, das als Komplement wirksam ist. Die Verdünnung wird mit einem Veronal-Puffer, enthaltend $MgCl_2$ und $CaCl_2$ (Kabat und Mayer 1964 a.a.O.) hergestellt. Man läßt die Mischung 2 Stunden bei Zimmertemperatur stehen und wäscht anschließend dreimal mit der vorhergenannten Waschlösung. Danach werden in die entleerten Gefäße 0,1 ml eines Anti-Human C 3 -Globulins, welches mit alkalischer Phosphatase nach der Methode von E.Engvall und P.Perlmann , Z. Immunol 109, (1972), S.129-135 konjugiert wurde, zugegeben. In einem Vorversuch wurde ermittelt, daß die geeignete Verdünnung dieses Reagenzes 1:200 mit phosphatgepufferter Kochsalzlöcung und einem Zusatz von 0,05% Tween 20 ist. Man läßt diese Mischung 3 Stunden bei Zimmertemperatur stehen, wäscht 2 mal mit der o.g. Waschlösung und gibt in jedes Gefäß 0,2 ml der Substratlösung für die alkalische Phosphatase. Sie enthält 1 mg Paranitrophenylphosphat/ml in 0,05 ml $Na_2CO_3$-Puffer pH 9,8 und $10^{-3}$ M $MgCl_2$. Nach 20 Minuten bei Zimmertemperatur wird 0,1 ml einer 0,1 M NaOH zugegeben. Damit wird die Reaktion gestoppt. Die quantitative Bestimmung

0009198

des gespaltenen Paranitrophenylphosphats erfolgt photometrisch bei einer Wellenlänge von 405 nm. Vergleichsansätze mit Normalseren sowie positiven Referenzseren
können mitgeführt werden.

PATENTANSPRÜCHE:

1. Verfahren zum Nachweis und zur Bestimmung eines komplementbindenden Antikörpers durch dessen Umsetzung mit einem Bindungspartner und Komplement, dadurch gekennzeichnet, daß man einen Bindungspartner des komplementbindenden Antikörpers unlöslich macht, den unlöslich gemachten Bindungspartner mit dem komplementbindenden Antikörper sowie Serumkomplement zur Umsetzung bringt, das Reaktionsprodukt mit einem Kupplungsprodukt eines Reaktionspartners des Serumkomplements und mit einem quantitativ bestimmbaren Markierungsmittel umsetzt und schließlich das Markierungsmittel bestimmt.

2. Verfahren nach Anspruch 1, dadurch gekennzeichnet, daß der Bindungspartner des komplementbindenden Antikörpers im Inneren eines Reaktionsröhrchens oder an der Oberfläche eines Trägers unlöslich gemacht ist.

3. Verfahren nach einem der Ansprüche 1 oder 2, dadurch gekennzeichnet, daß als Reaktionspartner des Serumkomplements ein Antikörper gegen einen Komplementfaktor verwendet wird.

4. Verfahren nach einem der Ansprüche 1 bis 3, dadurch gekennzeichnet, daß das Markierungsmittel ein Enzym ist.

5. Reagenz zur Durchführung des Verfahrens nach einem der Ansprüche 1 bis 4, gekennzeichnet durch einen Gehalt an
   a) trägergebundenem Antigen,
   b) Serumkomplement,
   c) Komplementfaktorantikörper verbunden mit einem Markierungsmittel.

Europäisches
Patentamt

**EUROPÄISCHER RECHERCHENBERICHT**

0009198

Nummer der Anmeldung

EP 79 10 3381

| Kategorie | Kennzeichnung des Dokuments mit Angabe, soweit erforderlich, der maßgeblichen Teile | betrifft Anspruch |
|---|---|---|
| A | DE - B - 2 037 507 (BAXTER LABORATORIES)<br>  * Ansprüche 1,2 * | 1 |
| | -- | |
| P | ARZNEIMITTEL FORSCHUNG, Band 28, Nr. 11A, November 1978, Seiten 1934-1940<br>Aulendorf, DE.<br>L. SCHROTT: "Enzymimmunoassay"<br>  * Seite 1936, Spalte 2; Absätze 1,2; Abbildung 4 * | 4,5 |
| | -- | |
| A | US - A - 3 654 090 (A.H.W.M. SCHUURS et al.)<br>  * Ansprüche 1,7,9 * | 4,5 |
| | ---- | |

## EINSCHLÄGIGE DOKUMENTE

**KLASSIFIKATION DER ANMELDUNG (Int.Cl. 3)**

G 01 N 33/54

**RECHERCHIERTE SACHGEBIETE (Int. Cl. 3)**

G 01 N 33/16
33/54
33/58

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 12-12-1979 | MEYLAERTS |

EPA form 1503.1  06.78